Europäisches Patentamt

European Patent Office

Office européen des brevets

(11) Publication number: **0 549 027 B1**

# EUROPEAN PATENT SPECIFICATION

(45) Date of publication of patent specification: **15.11.95** (51) Int. Cl.⁶: **A61K 7/16**

(21) Application number: **92203790.8**

(22) Date of filing: **07.12.92**

(54) **Mouthwash composition.**

(30) Priority: **11.12.91 GB 9126305**

(43) Date of publication of application:
**30.06.93 Bulletin 93/26**

(45) Publication of the grant of the patent:
**15.11.95 Bulletin 95/46**

(84) Designated Contracting States:
**AT BE CH DE DK ES FR GB GR IE IT LI NL PT SE**

(56) References cited:
**EP-A- 0 216 189**
**US-A- 4 937 066**

(73) Proprietor: **UNILEVER N.V.**
**P.O. Box 760**
**NL-3000 DK Rotterdam (NL)**

(84) Designated Contracting States:
**BE CH DE DK ES FR GR IT LI NL PT SE AT**

(73) Proprietor: **UNILEVER PLC**
**Unilever House**
**Blackfriars**
**P.O. Box 68**
**London EC4P 4BO (GB)**

(84) Designated Contracting States:
**GB IE**

(72) Inventor: **Saxton, Charles Alan, Unilever Research**
**Port Sunlight Lab.,**
**Ouarry Road East,**
**Bebington**
**Wirral,**
**Merseyside L63 3JW (GB)**
Inventor: **Klugkist, Jan, Unilever Research**
**Port Sunlight Lab.,**
**Ouarry Road East,**
**Bebington**
**Wirral,**
**Merseyside L63 3JW (GB)**
Inventor: **Grigor, Janet, Unilever Research**
**Port Sunlight Lab.,**
**Ouarry Road East,**
**Bebington**
**Wirral,**
**Merseyside L63 3JW (GB)**

(74) Representative: **van Gent, Jan Paulus**
**Unilever N.V.,**
**Patent Division**
**Postbus 137**
**NL-3130 AC Vlaardingen (NL)**

**Description**

The present invention relates to mouthwash compositions having anti-plaque efficacy. More particularly it relates to anti-plaque mouthwash compositions that contain a zinc salt and Triclosan as the anti-plaque system.

Various mouthwash compositions have been proposed in the art, mainly for the purpose of giving a feeling of freshness in the mouth. Although sometimes also a claim to a certain anti-plaque efficacy is made, nearly none of the mouthwash compositions that are on the market do show any significant anti-plaque benefits. One of the very few products with some anti-plaque benefits is Listerine, a product marketed by Warner Lambert, which consists of a mixture of thymol, hexylresorcinol, menthol, eucalyptol and methylsalicylate in a rather high amount of alcohol; others include Peridex marketed by Procter and Gamble, and Corsodyl marketed by ICI, which are based on a solution of chlorhexidine.

Thus, despite the many proposals for mouthwash compositions, only very few that have an anti-plaque benefit have reached the market, and there still exists the need for an effective anti-plaque mouthwash composition.

The combination of a zinc salt and Triclosan is a well-known, effective anti-plaque system, and toothpastes with this anti-plaque system are widely marketed. This system has also been proposed for inclusion in a mouthwash composition, but either the levels thereof are too high for a truly acceptable product, or such compositions suffer from other drawbacks like unpleasant taste, cloudy solutions, insufficient storage stability and the like.

We have now found that effective, clear mouthwash compositions with a zinc salt and Triclosan can be obtained if the compositions contain a nonionic surface-active agent and contain not more than 60% by weight of water, the compositions further containing a lower aliphatic monohydric alcohol and a humectant.

Thus, in its broadest aspect the present invention provides a mouthwash composition comprising a zinc salt and 2',4,4'-trichloro-2-hydroxy-diphenylether (Triclosan) as an anti-plaque system, a lower aliphatic monohydric alcohol selected from ethanol, isopropanol or mixtures thereof and a humectant, and a surface-active agent, characterised in that the surface-active agent comprises a nonionic surface active agent which is an ethylene oxide/propylene oxide block copolymer of the general formula H-(O-CH2CH2)a-(O-CH-(CH3)-CH2)b-(O-CH2CH2)a-OH in which a and b are integers greater than 0, said copolymer having an average molecular weight of between 4,000 and 15,000 and having an HLB-value between 27 and 30.5 and comprising about 80% by weight of ethylene oxide in the molecule, or a hydrogenated castor oil, condensed with 40-60 moles of ethylene oxide, the composition comprising not more than 60% by weight of water.

The zinc salt that is used in the present invention can be any zinc salt that provides an effective amount of zinc$^{2+}$ ions in the oral cavity. Examples of such zinc salts are enumerated in USP 4,022,880 and typically suitable zinc salts are zinc phenolsulphonate, zinc sulphate, zinc glycinate, zinc citrate, zinc chloride, zinc acetate and so on. They may be used as such, or they may be formed in situ, e.g. zinc glycinate may be formed in situ from zinc sulphate and glycine. Mixtures of various zinc salts may also be used.

The amount of zinc salt used in the composition may vary from 100-4000 ppm, expressed as zinc ions and will usually range from 200-2000 ppm, preferably from 300-1000 ppm. Triclosan, which is 2',4,4'-trichloro-2-hydroxy-diphenyl ether, is used in an amount of 0.01-2%, usually 0.02-0.5% and preferably 0.1-0.3% by weight of the composition.

The lower aliphatic monohydric alcohol can be ethanol, isopropanol or mixtures thereof, and is used in an amount of 5-25%, preferably 7.5-20% and particularly preferably 10-15% by weight of the composition.

The humectant can be any well-known humectant, used in the art for oral compositions such as glycerol, sorbitol, propyleneglycol, polyethyleneglycol and mixtures thereof. The humectant is usually used in an amount of 5-50%, preferably 10-40% and especially preferably 20-40% by weight of the composition.

An essential element of the present invention is the presence in the mouthwash composition of a nonionic surface-active agent. The nonionic surface-active agent is basically a condensation product of alkylene oxides with a hydrophobic moiety which can be fatty alcohol, a fatty acid, fatty acid amide, a fatty acid ester, an alkylphenol and so on. Typical examples are the condensation products of ethylene oxide, propylene oxide, butylene oxide and mixtures thereof with $C_8$-$C_{18}$ primary or secondary, branched or straight-chain alcohols, $C_8$-$C_{18}$ fatty acid amides, $C_9$-$C_{18}$ alkylphenols, and block copolymers of ethyleneoxide and propyleneoxide. Further suitable examples can be found in M. Schick, "Nonionic Surfactants" 1967. Naturally, the nonionic surface-active agent should be suitable for use in oral products, and should meet the safety requirements for such use. Particularly suitable examples are the ethylene oxide/propylene oxide block copolymers of the general formula

2

$$H\text{-}(O\text{-}CH_2CH_2)_a\text{-}(O\text{-}CH(CH_3)CH_2)_b\text{-}(O\text{-}CH_2CH_2)_a\text{-}OH$$

in which a and b are integers greater than 0 which are commercially available from ICI under the trade name "Synperonic PE" or "Pluronic". Of these block copolymers particularly those, containing 80% by weight of ethylene oxide in the molecule are preferred. Such products have an approximate molecular weight ranging from abt. 4,000 to abt. 15,000, and have an HLB ranging from 27-30.5. Specific examples of these preferred products are Synperonic PE F38, F68, F88 and F108.

Another type of preferred nonionic surface-active agents is the class of alkoxylated fatty acid esters such as hydrogenated castor oil, condensed with ethylene oxide, e.g. hydrogenated castor oil, condensed with 40 or 60 moles of ethylene oxide, commercially available from BASF under the trade name Cremophor RH40 and RH60. Other suitable examples of nonionic surface-active agents include polyoxyethylene sorbitan monolaurate and polyoxyethylene sorbitan monooleate, known as Tween 20 and Tween 80, available from ICI. Mixtures of various nonionic surface-active agents may also be used.

The amount of nonionic surface-active agent, used in the present invention is relatively low and ranges from 0.3-5%, preferably from 0.5-2% and particularly preferably from 0.5-1% by weight of the final composition.

The water-content of the composition of the present invention is also rather critical; it must be below 60% by weight. Above this level the compositions are not effective and often tend to be not clear, cloudy and suffer from phase-separation. Preferably, the water content is below 50%. Although it would be possible to provide non-aqueous systems, for most practical purposes, and also for cost reasons, the systems usually contain water, the lower water level being in the order of magnitude of 20%. The composition of the present invention may furthermore contain optional other ingredients such as flavours, preferably in an amount of 0.1-0.2%, sweetening agents, colouring agents, polymers, thickening agents, enzymes, other anti-plaque agents, anti-caries agents such as sodium fluoride, anti-tartar agents, anti-sensitive teeth agents etc. Small amounts of anionic surface active agents such as alkalimetal alkyl sulphates may also be present. They may be in the form of simple liquids, or they may be in the form of gels, suitable for topical application.

The composition of the invention should have a pH of between 4 and 8, preferably between 5 and 7, the preferred pH being 6.

The compositions of the present invention can be manufactured by simply mixing the ingredients in any desired or convenient manner. Preferably the order of addition is such that salts which would increase the ionic strength of the solution are added at the final stage.

Thus, a preferred order of addition is first dissolving any flavour and the Triclosan in the aliphatic monohydric alcohol, then adding the surfactant (as a solution), subsequently adding the humectant and finally adding the salts.

The invention will now further be illustrated by way of Example.

Example 1

The following formulations were prepared by mixing the various ingredients, the salts being added last.

| | % by weight | | | |
| --- | --- | --- | --- | --- |
| | A | B | C | D |
| Triclosan | 0.05 | 0.05 | 0.15 | 0.15 |
| Zinc phenolsulphonate (octahydrate) | 0.70 | 0.70 | 0.70 | 0.70 |
| Ethanol | 15.0 | 15.0 | 15.0 | 15.0 |
| Sorbitol (70% syrup) | -- | -- | -- | 40.0 |
| Glycerol | 40.0 | -- | -- | -- |
| Polyethyleneglycol(MW400) | -- | 40.0 | 40.00 | -- |
| Cremophor RH 60 | 0.50 | 0.50 | 0.50 | -- |
| Synperonic PE F68 | -- | -- | -- | 1.00 |
| Flavour | 0.20 | 0.20 | 0.20 | 0.20 |
| Colouring agent | -- | 0.025 | 0.025 | 0.025 |
| Water | 43.55 | 43.525 | 43.425 | 54.925* |

* including the water, present in the sorbitol syrup, which is 12%.

All these formulations were clear, without any sediment formed.

These products were tested as to their anti-plaque efficacy. For that purpose the plaque growth inhibition (PGI) was determined as follows:

The effectiveness of the mouthwash compositions of this invention in inhibiting the growth of plaque on the teeth was determined by following a standard procedure for the measurement of plaque growth. The methodology of measuring plaque growth is that according to Harrap as described in J. Clin. Periodontol., 1974, 1, 166-174 which gives a procedure for assessing the amount of plaque on the teeth adjacent to the gingival margin. The procedure is as follows:

During the late afternoon each subject brushes his/her teeth with a simple, non-active toothpaste (placebo) (having a composition as given hereinafter) for an unspecified period of time to remove as much plaque as possible. This is immediately followed by rinsing with water. Subsequently, any remaining plaque is disclosed by applying an aqueous solution of Erythrosin (0.5% w/w) to the teeth using a soft camel hair brush. Excess dye is removed by rinsing with water and the amount of plaque assessed and recorded for each of 16 teeth (numbers 3 to 6 for each quadrant). The recorded plaque is designated $P_0$. Thereafter, the mouth is rinsed for one minute with 10 ml of the mouthwash to be tested.

No further oral hygiene is permitted for 18 hours after which time each subject rinses his/her mouth with water to remove food debris and viscous saliva. Plaque assessment is then carried out as before and recorded ($P_{18}$). The values of $P_{18}$ and $P_0$ for each tooth are averaged to give a $P_{18}$ and $P_0$ value per mouth. The mean of the values obtained for the subjects in the test is the plaque growth value. Panels of at least 12 subjects are used with each subject using each of the mouthwashes in a randomised block sequence. The plaque growth value for a product without active ingredients is usually in the range 22 to 26. The plaque growth inhibition (PGI) is then computed for each test treatment by expressing the percentage inhibition compared to placebo:

$$PGI = \frac{PG_{pl} - PG_T}{PG_{pl}} \times 100\%$$

wherein $PG_{pl}$ = recorded plaque of the placebo and $PG_T$ = recorded plaque of the test composition.

The composition of the simple, non-active toothpaste referred to above was the following:

| Ingredient | % |
|---|---|
| Alumina trihydrate | 50.00 |
| Glycerin | 27.00 |
| Hydroxyethylcellulose | 0.95 |
| Titanium dioxide | 0.50 |
| Water | to 100.00 |

Compositions A-D were assessed as to their PGI in accordance with the above test protocol, and were found to have the following PGI values

| A | B | C | D |
|---|---|---|---|
| 53% | 56% | 65% | 53% |

Products A and D were tested against a water "placebo" (containing 0.2% water-soluble flavour), and Products B and C against a water "placebo" containing 0.2% water-soluble flavour and 0.025% colouring agent.

Example 2

The following mouthwash composition were prepared:

% by weight

| | E | F | G | H | I | J | K** | Place-bo |
|---|---|---|---|---|---|---|---|---|
| Triclosan | 0.03 | 0.15 | 0.15 | 0.15 | 0.15 | - | - | - |
| Zinc phenol-sulphonate octahydrate | 0.70 | - | - | - | - | - | - | - |
| Zinc sulphate heptahydrate | - | 0.20 | 0.40 | 0.40 | - | 0.40 | - | - |
| Glycine | - | 0.5 | 1.8 | 1.8 | - | 1.8 | - | - |
| Ethanol | 7.8 | 6.0 | 10.0 | 15.0 | 15.0 | 15.0 | 11.6 | 15.0 |
| Sorbitol (70% syrup) | 7.8 | 7.0 | - | 40.0 | 40.0 | 40.0 | - | 40.0 |
| Glycerol | 16.3 | - | 10.0 | - | - | - | pre-sent | - |
| Propylene glycol | 7.4 | - | - | - | - | - | - | - |
| Gantrez S-97* | 0.25 | - | - | - | - | - | - | - |
| Synperonic PE F 68 | - | - | - | 1.0 | 1.0 | 1.0 | - | 1.0 |
| Cremophor RH 40 | - | 1.0 | 1.0 | - | - | - | - | - |
| Sodium lauryl-sulphate | 0.4 | 0.34 | - | - | - | - | - | - |
| Sodium lauroylsar-cosinate | 0.2 | - | - | - | - | - | - | - |
| Flavour | 0.2 | 0.2 | 0.2 | 0.2 | 0.2 | 0.2 | pre-sent | 0.2 |
| Water (incl. water stemming from the sorbitol syrup) | 61.22 | 86.71 | 76.45 | 53.45 | 55.65 | 53.60 | pre-sent | 55.80 |

* vinylmethylether/maleic anhydride copolymer ex GAF

** product K also contained 0.12% chlorhexidine digluconate

Products F, G, H and J were adjusted to pH 6.0 with NaOH.

The PGI values for these products compared with the placebo were as follows:

| E | F | G | H | I | J | K |
|---|---|---|---|---|---|---|
| 21% | 18% | 9% | 31% | 15% | 13% | 37% |

This clearly shows the improved benefit of a product according to the invention, product H, over products not according to the invention, products E, F and G, and demonstrates a synergistic action of zinc and Triclosan, comparing product H with products I and J. The product is almost as effective as product K. Product E without a zinc salt had a PGI of 16%.

Example 3

The following formulations were tested as to their PGI, using water + flavour as placebo.

Formulations:

| | L | M | N |
|---|---|---|---|
| Zinc phenolsulphonate 8H$_2$O | 0.70 | 0.30 | -- |
| Triclosan | 0.05 | 0.01 | -- |
| Ethanol | 15.00 | 10.00 | -- |
| Glycerol | 40.00 | 40.00 | -- |
| Flavour | 0.20 | 0.20 | -- |
| Cremophor RH 60 | 0.50 | 0.50 | -- |
| Chlorhexidinedigluconate | -- | -- | 0.1 |
| Water | 43.55 | 48.99 | 99.9 |
| PGI (%) | 60 | 33 | 67 |

Formulations M still showed satisfactory effects, despite the low levels of zinc (350 ppm Zn$^{2+}$) and Triclosan (0.01%). Formulation L with 0.7% ZPS (824 ppm Zn$^{2+}$) + 0.05% TCN was almost as effective as formulation N.

Example 4

The following formulations were tested as to their PGI, using water as placebo (formulation O).

| | O | P | Q | R | S | T |
|---|---|---|---|---|---|---|
| Triclosan | -- | 0.05 | 0.05 | 0.05 | 0.05 | 0.05 |
| PEG400 | -- | 40.0 | 40.0 | 40.0 | 40.0 | 40.0 |
| ethanol | -- | 15 | 10 | 5 | 0 | 15 |
| zincphenolsulphonate octahydrate | -- | 0.7 | 0.7 | 0.7 | 0.7 | 0.7 |
| cremophor RH 60 | -- | 0.5 | 0.5 | 0.5 | 0.5 | -- |
| flavour | 0.2 | 0.2 | 0.2 | 0.2 | 0.2 | -- |
| colour (1% soln) water to 100 | 0.025 | 0.025 | 0.025 | 0.025 | 0.025 | 0.025 |
| PGI effect (% inhibition) | placebo | 56% | 40% | 16% | 17% | 35% |

These results show, that the formulations should have ethanol levels higher than 5%, preferably more than 10%, and should contain a nonionic surfactant and flavour for highest efficacy.

Example 5

The following formulations were tested as to their PG1, using an ethanol/sorbitol mouthwash as placebo.

|  | A | B | Placebo |
|---|---|---|---|
| zinc sulphate 7H$_2$O | -- | 0.4% | -- |
| Glycine | -- | 1.8% | -- |
| Zincphenolsulphonate 8H$_2$O | 0.7% | -- | -- |
| Triclosan | 0.15% | 0.15% | -- |
| Ethanol | 15.0% | 15.5% | 15.0% |
| Sorbitol (70% syrup) | 40.0% | 40.0% | 40.0% |
| Synperonic PE/F68 | 1.0% | 1.0% | 1.0% |
| Flavour | 0.2% | 0.1% | 0.2% |
| Colouring agent (1% aq.) | 0.25% | 0.25% | 0.25% |
| Water (excl. sorbitol) | 42.7% | 41.3% | 41.2% |

The pH was adjusted with HCl and/or NaOH.

| Formulation | A | A | A | A | B |
|---|---|---|---|---|---|
| pH | 4.49 | 5.27 | 6.23 | 6.93 | 6.69 |
| PGI (% inhibition) | 17% | 25% | 34% | 25% | 24% |

These results show, that the pH for optimum anti-plaque effect should be from 5-7, preferably 6 and they also show that Zinc/glycinate (910 ppm zinc) is as effective as zincphenol sulphonate (825 ppm zinc).

Example 6

The following formulations were tested as to their PG1, using an ethanol mouthwash as placebo (u).

|  | U | V | W | X | Y | Z | AA |
|---|---|---|---|---|---|---|---|
| Triclosan | -- | 0.15 | 0.15 | 0.15 | 0.15 | 0.15 | 0.15 |
| Zinc sulphate heptahydrate | -- | 0.40 | 0.40 | 0.40 | 0.40 | 0.40 | -- |
| Glycine | 1.80 | 1.80 | 1.80 | 1.80 | 1.80 | 1.80 | -- |
| Glycerol | 10.00 | 10.00 | 10.00 | 10.00 | 10.00 | 10.00 | -- |
| Ethanol | 10.00 | 15.00 | 10.00 | 7.50 | 10.00 | 10.00 | 15.00 |
| Cremophor RH40 | 1.00 | 1.00 | 1.00 | 1.00 | 0.60 | 2.00 | 0.50 |
| Flavour | 0.20 | 0.20 | 0.20 | 0.20 | 0.20 | 0.20 | -- |
| Saccharin | 0.04 | 0.04 | 0.04 | 0.04 | 0.04 | 0.04 | -- |
| Colour (1% soln) | 0.75 | 0.75 | 0.75 | 0.75 | 0.75 | 0.75 | 0.25 |
| Zinc phenolsulphonate octahydrate | -- | -- | -- | -- | -- | -- | 0.7 |
| PEG 400 | -- | -- | -- | -- | -- | -- | 40.0 |
| Water to 100% | 76.21 | 70.66 | 75.66 | 78.16 | 76.06 | 74.66 | 43.40 |
| PGI | placebo | 2% | -8% | 16% | 9% | -2% | 53% |

These results show that product AA of the invention is significantly more effective than products U-Z which are not according to the invention.

**Claims**

1. A mouthwash composition comprising a zinc salt and 2',4,4'-trichloro-2-hydroxy-diphenylether (Triclosan) as an anti-plaque system, a lower aliphatic monohydric alcohol selected from ethanol, isopropanol or mixtures thereof and a humectant, and a surface-active agent, characterised in that the surface-active agent comprises a nonionic surface active agent which is an ethylene oxide/propylene oxide block copolymer of the general formula H-(O-CH2CH2)a-(O-CH-(CH3)CH2)b-(O-CH2CH2)a-OH in

7

which a and b are integers greater than 0, said copolymer having an average molecular weight of between 4,000 and 15,000 and having an HLB-value between 27 and 30.5 and comprising about 80% by weight of ethylene oxide in the molecule, or a hydrogenated castor oil, condensed with 40-60 moles of ethylene oxide, the composition comprising not more than 60% by weight of water.

2. A composition according to claim 1, characterized in that it contains from 20-50% by weight of water.

3. A composition according to claim 1 or 2, characterized in that it comprises from 100-4000 ppm, expressed as zinc ions, of the zinc salt, from 0.01-2% by weight of Triclosan, from 5-25% by weight of the lower aliphatic monohydric alcohol, from 5-50% of the humectant and from 0.3-5% by weight of the nonionic surface-active agent.

4. A composition according to claims 1-3, characterized in that the zinc salt is zinc phenolsulphonate or zinc glycinate.

## Patentansprüche

1. Mundwasserzusammensetzung, umfassend ein Zinksalz und 2',4,4'-Trichlor-2-hydroxydiphenylether (Triclosan) als Antiplacuesystem, einen niederaliphatischen einwertigen Alkohol, ausgewählt aus Ethanol, Isopropanol oder Gemischen davon und ein Feuchthaltemittel und ein Tensid, dadurch gekennzeichnet, daß das Tensid ein nichtionisches Tensid, das ein Ethylenoxid/Propylenoxidblockcopolymer der allgemeinen Formel H-(O-CH$_2$CH$_2$)$_a$-(O-CH-(CH$_3$)CH$_2$)$_b$-(O-CH$_2$CH$_2$)$_a$-OH ist, worin a und b ganze Zahlen größer 0 darstellen, wobei das Copolymer ein mittleres Molekulargewicht zwischen 4000 und 15000 und einen HLB-Wert zwischen 27 und 30,5 aufweist und etwa 80 Gew.-% Ethylenoxid in dem Molekül umfaßt, oder ein hydriertes Rizinusöl umfaßt, kondensiert mit 40 bis 60 Mol Ethylenoxid, wobei die Zusammensetzung nicht mehr als 60 Gew.-% Wasser umfaßt.

2. Zusammensetzung nach Anspruch 1, dadurch gekennzeichnet, daß sie 20 bis 50 Gew.-% Wasser enthält.

3. Zusammensetzung nach Anspruch 1 oder 2, dadurch gekennzeichnet, daß sie 100 bis 4000 ppm, ausgedrückt in Zinkionen des Zinksalzes, 0,01 bis 2 Gew.-% Triclosan, 5 bis 25 Gew.-% niederaliphatischen einwertigen Alkohol, 5 bis 50 % Feuchthaltemittel und 0,3 bis 5 Gew.-% nichtionisches Tensid umfaßt.

4. Zusammensetzung nach den Ansprüchen 1 bis 3, dadurch gekennzeichnet, daß das Zinksalz Zinkphenolsulfonat oder Zinkglycinat ist.

## Revendications

1. Composition de collutoire comprenant un sel de zinc et l'éther 2',4,4'-trichloro-2-hydroxydiphénylique (Triclosan) en qualité de système anti-plaque, un monoalcool aliphatique inférieur choisi parmi l'éthanol, l'isopropanol ou des mélanges de ceux-ci et un humectant et un agent tensioactif, caractérisée en ce que l'agent tensioactif comprend un agent tensioactif non ionique qui est un copolymère séquencé oxyde d'éthylène/oxyde de propylène de formule générale :

H-(O-CH$_2$CH$_2$)a-(O-CH-(CH$_3$)CH$_2$)b-(O-CH$_2$CH$_2$)a-OH

dans laquelle a et b sont des nombres entiers supérieurs à 0, ledit copolymère ayant une masse moiéculaire moyenne comprise entre 4000 et 15.000 et un indice d'amphipathie de 27 à 30,5 et comprenant environ 80% en poids d'oxyde d'éthylène dans la molécule, ou une huile de ricin hydrogénée condensée avec 40 à 60 moles d'oxyde d'éthylène, la composition ne comprenant pas plus de 60% en poids d'eau.

2. Composition selon la revendication 1, caractérisée en ce qu'elle contient de 20 à 50% en poids d'eau.

3. Composition selon la revendication 1 ou 2, caractérisée en ce qu'elle comprend de 100 à 4000 ppm, exprimées en ion zinc, de sel de zinc, de 0,01 à 2% en poids de Triclosan, de 5 à 25% en poids d'un monoalcool aliphatique inférieur, de 5 à 50% d'humectant et de 0,3 à 5% en poids de tensioactif non ionique.

4. Composition selon les revendications 1 à 3, caractérisée en ce que le sel de zinc est le phénosulfonate de zinc ou le glycinate de zinc.